# EUROPEAN PATENT SPECIFICATION

(11) **EP 3 463 155 B1**
(45) Date of publication and mention of the grant of the patent: **07.02.2024**
(21) Application number: 17725763.1
(22) Date of filing: 23.05.2017
(51) Int. Cl.: A61B 34/30, A61B 18/14, A61B 90/00, A61B 34/20, A61B 90/50

(54) **SURGICAL INSTRUMENT, ROBOTIC ARM AND CONTROL SYSTEM FOR A ROBOTIC ARM**
CHIRURGISCHES INSTRUMENT, ROBOTERARM UND STEUERUNGSSYSTEM FÜR EINEN ROBOTERARM
INSTRUMENT CHIRURGICAL, BRAS ROBOTISÉ ET SYSTÈME DE COMMANDE DE BRAS ROBOTISÉ

(30) Priority: 23.05.2016 GB 201608997; 23.05.2016 GB 201609030; 23.05.2016 GB 201609002
(43) Date of publication of application: 10.04.2019
(73) Proprietor: IP2IPO Innovations Limited, London N1C 4AG (GB)
(72) Inventor: YANG, Guang-Zhong, London SW7 2AZ (GB); WISANUVEJ, Piyamate, London SW7 2AZ (GB); LEIBRANDT, Konrad Marek Günter, London SW7 2AZ (GB); SENECI, Carlo Alberto, London SW7 2AZ (GB); SHANG, Jianzhong, London SW7 2AZ (GB); LIU, Jindong, London SW7 2AZ (GB)
(74) Representative: Potter Clarkson
(86) International application number: PCT/GB2017/051436
(87) International publication number: WO 2017/203231

(56) References cited:
- US-A1- 2006 199 999
- US-A1- 2008 058 861
- US-A1- 2012 215 220
- US-A1- 2014 257 331
- US-B1- 9 033 998

## Description

### FIELD

The present invention provides a surgical instrument.

### BACKGROUND

Traditional laparoscopic manual instruments are composed of a handle, a rigid shaft and a functional end effector, such as graspers, scissors or suction channels for example. Usually, two laparoscopic instruments are used at the same time by a surgeon. The laparoscopic instruments may be located within a single port or within multiple ports. The common characteristics of all these instruments are that motion is transmitted from the handle to the end effector by exploiting the fulcrum effect between the rigid shaft and the port where the instrument is inserted. Generally, instruments used in laparoscopic surgery provide four degrees of freedom. Taking transanal endoscopic micro-surgery as an example, the workspace available to a surgeon is very limited meaning that manoeuvring the handles of prior art instruments to achieve the fulcrum effect is very challenging and instrument collision is common both at the functional end effector and handle.

Manual articulated laparoscopic surgical tools are inherently bulky and provide challenges to surgeons in terms of safely using such tools within a limited workspace. A large amount of research has been undertaken into robotic surgical tools for use in many different medical applications. Examples are:
CN104434318 describes an example of a robotic surgical instrument that provides four degrees of freedom.
K100778387 describes a surgery robot for laparoscopic procedures that comprises a hinged elbow function and a rotatable wrist function.
US5624398 describes an endoscopic robotic surgical tool that provides a shoulder flexion joint, upper arm rotational joint, elbow flexional joint and wrist rotational joint.
US8603135 describes an example of an articulating surgical instrument constructed from a series of links to enable snake-like motion of the surgical instrument.

However, prior art robotic articulated surgical tools are not suitable for use in laparoscopic procedures where space is limited. The prior art robotic articulated surgical tools also do not have sufficient DoF at the tool tip or suitably sized tool tips for use in many laparoscopic procedures.

During surgery, a surgeon is constrained to working within a tightly defined workspace. It is important that the surgeon does not permit surgical instruments to deviate from within the defined workspace or damage or injury could result to a patient. Measures are therefore required to prevent surgical instruments from deviating from the defined workspace.

US2005/0166413 describes a robotic arm that can define a boundary prior to use by moving the arm through a pre-determined set of co-ordinates. In use, if the boundary is crossed the arm is disabled to prevent further movement outside of the boundary.

US2010174410 describes a robotic arm that is operated by depression of a single operating switch.

US9033998 B1 describes a robotic system for use in minimally invasive surgery that comprises a robotic arm. The robotic arm is provided with an independent roll wrist mechanism, which can allow for imparting the roll motion to end effectors while mitigating the need of rotating the entire robotic arm, thus ensuring independent movement of end effectors.

US2014/257331 A1 describes a laparoscopic surgical devices include a first member having a first gear, a second member having a second gear corresponding to the first gear, a connection member configured to connect the first member and the second member; first and second wire mounting pieces respectively at the first and second members, a first wire wound on the first wire mounting pieces so as to be pulled upon receiving a first drive force; and a second wire wound on the second wire mounting pieces so as to be pulled upon receiving a second drive force. The drive unit is configured to selectively transmit the first and second amplified driving forces to a respective one the first member and the second member to cause tilting of the first member and the second member.

US2008/058861 A1 describes a surgical instrument body having a proximal portion, a distal portion, and a joint between the proximal and distal instrument body portions. A drive element housing extends through the proximal and distal instrument body portions and through the joint. A force to actuate a component at the distal end of the instrument body is applied to the drive element. A second force in the opposite direction is applied to the drive element housing, and this second force is also applied to the component. The opposite direction forces stabilize the component so that when the distal component is actuated, the actuation does not significantly affect the joint position.

US2006/199999 A1 describes an articulate minimally invasive surgical instrument with a flexible wrist to facilitate placement and provide visual verification of an ablation catheter or other devices in Cardiac Tissue Ablation (CTA) treatments. In one embodiment, the instrument is an endoscope which has an elongate shaft, a flexible wrist at the working end of the shaft, and a vision scope lens at the tip of the flexible wrist.

US2012/215220 A1 describes a surgical instrument including a shaft having a proximal end and a distal end, and a wrist coupled to the distal end of the shaft and configured to articulate in multiple degrees of freedom coupled to the distal end of the shaft. The surgical instrument can further include an end effector supported by the wrist, wherein the end effector includes a cutting element and jaws configured to grip tissue and fuse tissue via electrosurgical energy.

Robotic surgery typically involves the use of a port device mounted on a robotic arm.

The port device comprises a limited number of lumens for receiving respective surgical tools. Often, surgeons utilise all ports in the port device and require additional tools which have to be used independently of the port device.

The present invention seeks to overcome challenges encountered during transanal robotic endoscopic micro-surgery.

### SUMMARY OF THE INVENTION

The invention is defined in appended independent claim 1, further embodiments are described in the dependent claims.

An aspect of the invention provides a surgical instrument comprising: a rigid shaft, at least one elbow joint, itself comprising at least three elbow joints, hingedly coupled to the rigid shaft and a wrist joint coupled to the at least one elbow joint, wherein the wrist joint is configured to provide a first degree of freedom of movement and a second degree of freedom of movement, wherein the second degree of freedom of movement is substantially perpendicular to the first degree of freedom of movement, and
wherein two of the at least three elbow joints are arranged to provide a hinged motion in the same direction and a third elbow joint is arranged to provide a hinged motion in a different direction to the other elbow joints and wherein at least two adjacent elbow joints are coupled together such that the at least two adjacent elbow joints move in unison; and
wherein the wrist joint comprises first and second sections and an end effector having first and second jaws, the first section defining a first hinged joint with the second section so that the second section is hingedly moveable relative to the first section and the at least one elbow joint to provide the first degree of freedom of movement of the wrist joint, the second section defining a second hinged joint with each of the first and second jaws of the end effector so that each jaw is hingedly moveable relative to the first and second sections and the at least one elbow joint, the second hinged joint being arranged perpendicular to the first hinged joint so as to provide the second degree of freedom of movement of the wrist joint.

Provision of a surgical instrument with both an elbow joint and a wrist joint is advantageous as this configuration provides a surgeon with at least five degrees of freedom of movement. The rigid shaft transmits linear translation and axial rotation. The at least one elbow joint is connected to the rigid shaft and provides hinged motion between the at least one elbow joint and the wrist joint. The wrist joint provides both hinged and pivoting motion. Such a surgical instrument provides a surgeon with a greater range of motion within a restricted workspace than is possible in the prior art and provides a robotically controlled toolbox having all of the tools used by a surgeon in a conventional manual tool kit for laparoscopic surgery.

The surgical instrument may further comprise one or more additional elbow joints movable independently of any other elbow joint.

In another embodiment, the surgical instrument further comprises a bipolar or monopolar end effector.

Provision of a bipolar end effector confers a further degree of freedom of movement to the surgical instrument.

In embodiments of the invention, the end effector is moveable relative to first and second sections of the wrist joint by tendon drive means.

The tendon drive means may be shrouded by Bowden cables.

In embodiments of the invention the end effector is coupled to the at least one elbow joint, wherein the rigid shaft and the at least one elbow joint define a continuous lumen therethrough, the continuous lumen receiving an auxiliary end effector or providing irrigation or suction functionality.

The surgical instrument may further comprise a multi lumen insert positioned within the continuous lumen, the multi lumen insert comprising a plurality of lumens, wherein one or more of the plurality of lumens is configured to receive a respective tendon.

The surgical instrument may further comprise a protective sleeve, the protective sleeve comprising: an elongate flexible sheath having a first end and a second end, wherein the first end comprises an attachment means for attachment of the protective sleeve to the surgical instrument and wherein the second end comprises a closure means.

The attachment means may comprise a locking means having a first part located at the first end of the protective sleeve and a second part forming part of a robotic surgical system.

The attachment means may comprise a magnetic means having a first part located at the first end of the protective sleeve and a second part forming part of a robotic surgical system.

The flexible sheath may be compressible.

The closure means may be a valve.

The valve may be attached to the second end of the elongate sheath by way of a magnetic attachment means.

### FIGURES

The invention will now be described by way of reference to the following figures:
Figure 1 shows a surgical instrument according to aspects of the invention;
Figure 2 shows a first and second section of the surgical instrument of figure 1;
Figure 3 shows an illustrative view of the degrees of freedom of movement of the surgical instrument of figure 1;
Figure 4 shows a further view of the surgical instrument of figure 1;
Figure 5 shows a PTFE catheter for use with embodiments of the invention;
Figure 6 shows an example surgical instrument combining a primary end effector (bipolar) and suction and/or irrigation functionality;
Figure 7 shows an instrument base for coupling a surgical instrument to a robotic arm assembly;
Figure 8 shows a robotic arm;
Figure 9 shows a schematic of a control system for robotic systems;
Figure 10 shows a view of a protective sleeve for use with embodiments of the invention;
Figure 11 shows a detailed view of the protective sleeve of figure 10;

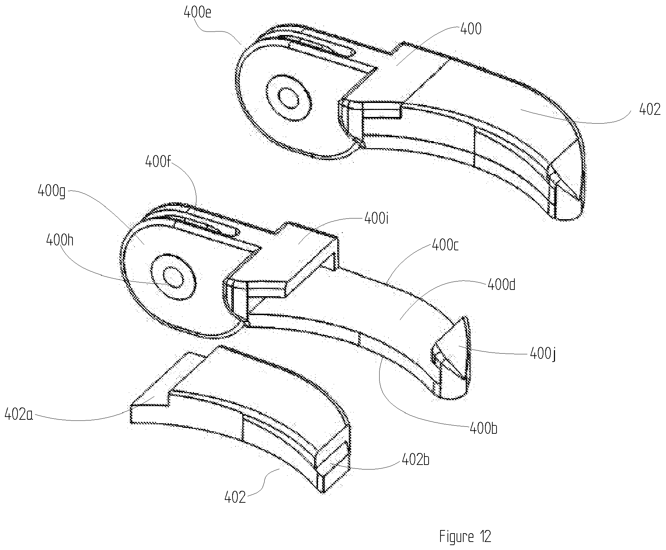

Figure 12 shows a view of an end effector adapted to receive a sensor therein;
Figure 13 shows a first view of a needle driver end effector;
Figure 14 shows a second view of the needle driver of figure 13;
Figure 15 shows an alternative embodiment of a needle driver;
Figure 16 shows a side view of an end effector with axial rotation imparted at the end effector.

### DESCRIPTION

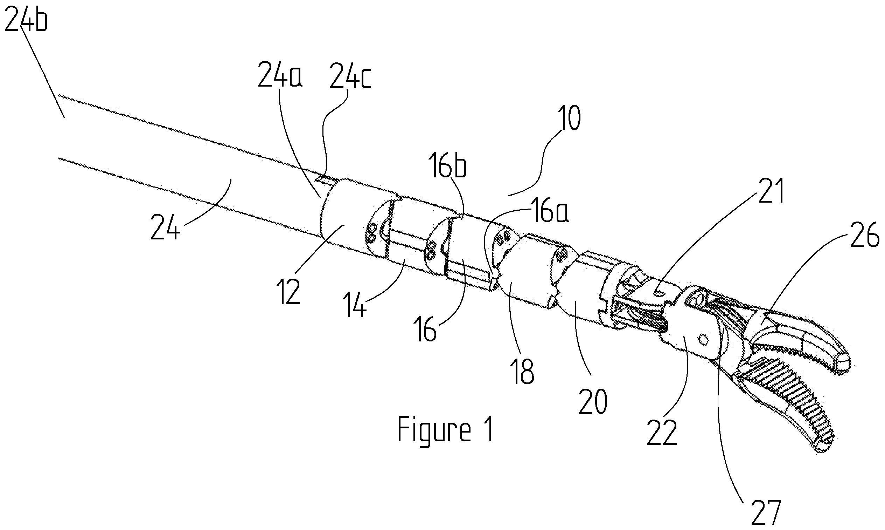

Surgical instruments according to aspects of the invention are illustrated generally in figure 1. A surgical instrument (10) comprises a plurality of sections (12, 14, 16, 18, 20, 22) connected to a rigid shaft (24). The rigid shaft (24) is connected to an instrument base (not shown in figure 1). An instrument tip (26), also referred to as an end-effector herein, is connected to the section (22) furthest away from the rigid shaft (24).

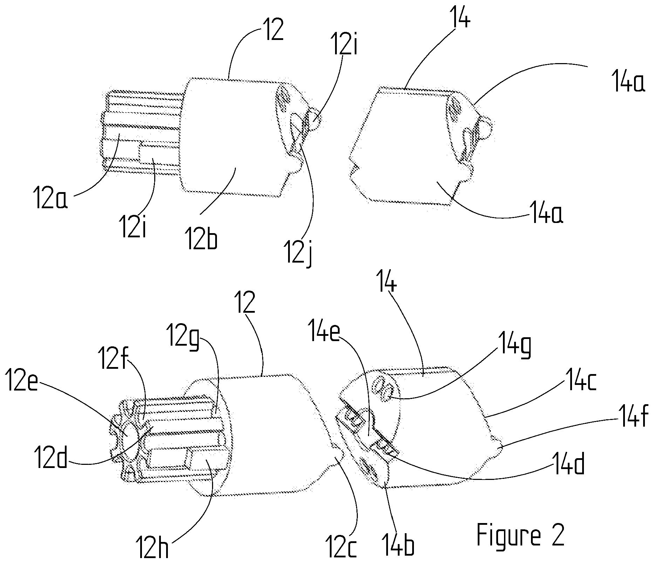

A first section (12), as illustrated in figure 2, is fixedly connected to the rigid shaft (24) by way of a splined connection (12a). The first section (12) comprises a generally cylindrical body (12b) having the splined connection (12a) at one end thereof and a mounting feature (12c) at the other end thereof. The splined connection (12a) is 4mm long and comprises eight projections (12d) extending radially from a central lumen (12e). Each of the eight projections (12d) are evenly spaced apart with a length of 1.7mm measured from the central axis of the first section (12) and define a scallop (12f) between each adjacent pair of the eight projections (12d). Each scallop (12f) receives a tendon (not shown in figures 2a and 2b) with each tendon passing through the generally cylindrical body (12b) of the first section (12) through a respective hole (12g) arranged around the central lumen (12e). The splined connection (12a) further comprises a locking formation (12h) for restricting or preventing rotation of the first section (12) relative to the rigid shaft (24).

The central lumen (12e) has a cylindrical profile and an internal diameter of between 1.5mm and 3mm.

The mounting feature (12c) comprises an opposite pair of generally semi-circular tabs (12i) extending longitudinally away from the generally cylindrical body (12b). Each generally semi-circular tab (12i) has a radius of 0.5m and a thickness of between 0.5mm and 1.5mm. The generally semi-circular tabs (12i) are mounted at the extreme end of the body (12b) and define between them a flattened apex (12j) from which the generally cylindrical body (12b) is chamfered in both directions away from the end of the first section (12) to enable relative movement of an adjacent section (14). The angle of chamfer in each direction is ninety four degrees to enable the adjacent section (14) to hingedly rotate through eighty degrees relative to the first section (12).

The rigid shaft (24), as shown in figure 1, comprises a hollow tube having an outer diameter of 5 mm and an inner diameter of 4 mm. The rigid shaft (24) is formed from stainless steel and is between 200mm and 300mm long. The first end (24a) of the rigid shaft (24) is configured to receive the splined connection (12a) of the first section (12) and restrict rotation of the splined connection (12a) of the first section (12) therein. The rigid shaft (24) is connected at the second end (12b) thereof to an instrument base (not shown in figure 1 or figure 2). The rigid shaft (24) is used to transmit linear translation and axial rotation motion from the instrument base to the end effector (26). All other degrees of freedom are controlled through use of the tendons that pass through the rigid shaft (24) to the surgical instrument sections (12, 14, 16, 18, 20, 22).

The rigid shaft (24) further comprises a complimentary locking formation (24c) for cooperation with locking formation (12h) of the first section (12) to prevent rotation of the first section (12) relative to the rigid shaft (24).

The second section (14), as illustrated in figure 2, is hingedly connected to the first section (12). The second section (14) comprises a generally cylindrical body (14a) having a first end (14b) and a second end (14c). The first end (14b) of the second section (14) comprises a groove of triangular cross section (14d) for receiving the generally semi-circular tabs (12i) of the mounting formation (12c) of the first section (12). The profile of the cylindrical body (14a) of the second section (14) is chamfered away from the triangular groove (14d) in both directions towards the second end (14c). The angle of chamfer in each direction is ninety four degrees to enable relative hinged movement between the first section (12) and the second section (14). The second section (14) further comprises an internal lumen (14e) substantially similar to the internal lumen (12e) of the first section (12).

The second end (14c) of the second section (14) comprises a mounting feature (14f) substantially the same as the mounting feature (12c) of the first section (12). A plurality of holes (14g) for receiving respective tendons pass longitudinally through the cylindrical body (14a) and surround the lumen (14e).

The third and fourth sections (16, 18) are substantially the same as the second section (14) and connected together in a snake like formation. The sections (12, 14, 16, 18) can be arranged to provide hinged movement in any direction as necessary according to intended use of the surgical instrument (10). The second section (14) as illustrated in figure 2 shows the mounting formation (14e) and triangular groove (14d) aligned. In other embodiments, such as illustrated in figure 1, the mounting formation (16a) and triangular groove (16b) are orientated at ninety degrees from one another. It will be appreciated that the orientation of the mounting formation (16a) and triangular groove (16b) can be selected based on the range of motion required for a particular application.

In some examples disclosed herein, each of the second, third and fourth sections (14, 16, 18) are movable independently of one another to provide maximum dexterity. However, embodiments of the invention require less dexterity and two or more adjacent sections are locked together causing such sections to move in unison.

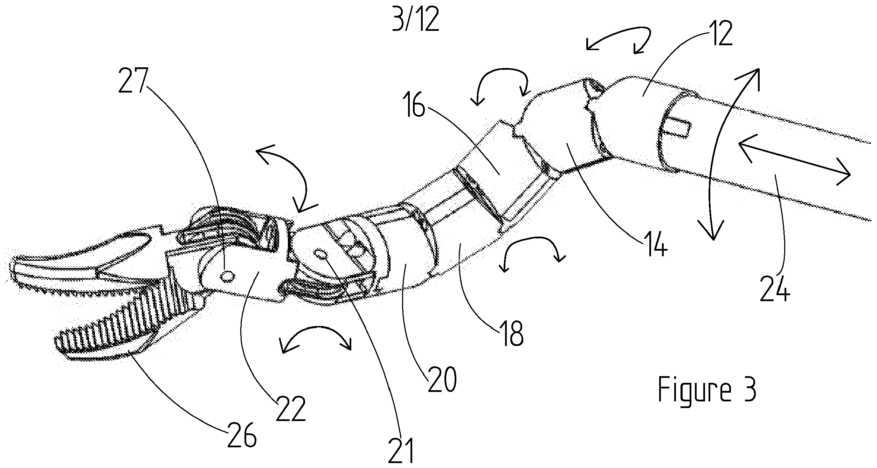

Figure 3 illustrates the degrees of freedom of movement of a surgical instrument (10) according to aspects of the invention. The arrows shown indicate the general direction of movement of each component of the surgical instrument (10).

In one embodiment the rigid shaft (24) imparts translational movement and axial rotation to the surgical instrument (10). None of the sections (12, 14, 16, 18, 20, 22) or end effector (26) have the independent ability to translate or rotate around the axis of the surgical instrument (10). The first section (12) is positionally fixed relative to the rigid shaft (24). The second section (14) defines an elbow joint with the first section (12) and is hingedly movable relative to the first section (12) through an angular range of movement of eighty degrees. The third section (16) defines an elbow joint with the second section (14) and is hingedly movable relative to the second section (14) through an angular range of movement of eighty degrees. The fourth section (18) defines an elbow joint with the third section and is hingedly movable relative to the third section (16) through an angular range of movement of up to eighty degrees. In some embodiments the angular range of movement is sixty degrees.

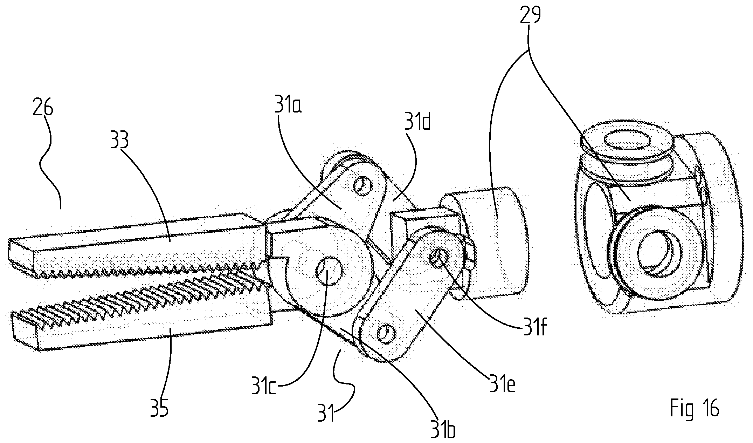

In another example, not according to the invention, axial rotation is imparted into the end effector (26) by an axial rotational joint (29), as shown in figure 16, The axial rotational joint (29) allows for two hundred and seventy degree axial rotation of the end effector (26). Axial rotation is imparted by way of a pair of tendons (not shown).

As illustrated in figure 16, an instrument comprising an axial rotational joint (29) adjacent to or integral with the end effector (26) further comprises a quadrilateral actuation mechanism (31) for opening and closing the jaws (33, 35). The quadrilateral mechanism comprises first and second arms (31a, 31b) connected to each of the jaws (33, 35) by a common pivot point (31c) and third and fourth arms (31d, 31e) respectively pivotally connected to the first and second arms (31a, 31b) and at a common pivot point (31f) acting as an anchor point for a drive tendon (31g). The drive tendon (31g) is operatively connected to a return spring (not shown) such that the jaws (33, 35) are biased in a closed configuration by the return spring.

The fifth section (20) and sixth section together define part of the wrist joint of the surgical instrument (10). The fifth section (20) defines an elbow with the fourth section (18) and is hingedly movable relative to the fourth section (18). The fifth section (20) also defines a separate hinged joint (21) with the sixth section (22). The sixth section (22) is hingedly movable relative to the fifth section (20). The sixth section (22) defines a hinged connection (27) with an end effector (26) which is arranged perpendicular to the hinged connection between the fifth section (20) and sixth section (22). The hinged connection (27) between the sixth section (22) and end effector (26) and the hinged connection (21) between the fifth section (20) and sixth section (22) together define all DoF provided by the wrist joint.

In some examples disclosed herein, each of the sections (14, 16, 18, 20, 22) is independently movable relative to adjacent sections (14, 16, 18, 20, 22). This arrangement enables the surgical instrument (10) to be manoeuvred in a snake like manner to provide an optimised motion path for a surgeon during surgery. However, in embodiments of the invention, sections (14, 16, 18, 20) may be coupled to adjacent sections (12, 14, 16, 18, 20) such that one or more adjacent sections (14, 16, 18, 20) move together.

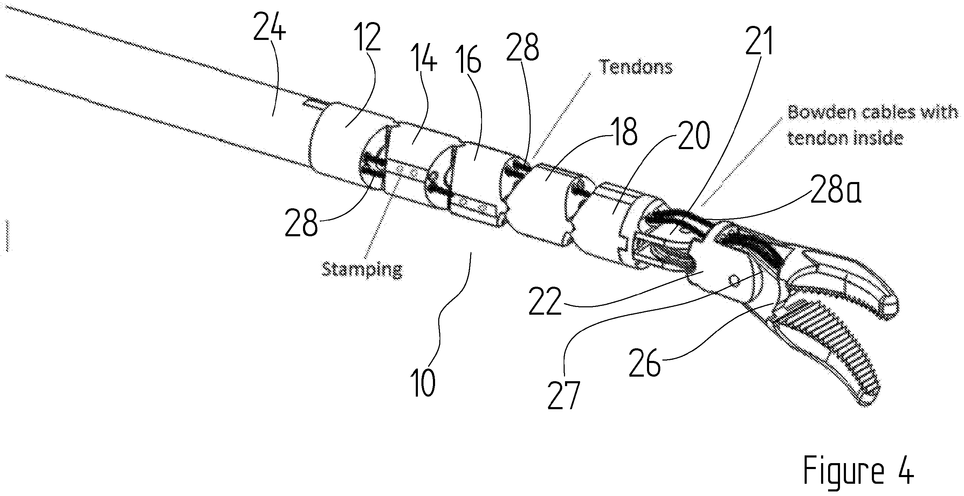

As shown in figure 4, tendons (28) passing through the lumen in the rigid shaft (24) and through respective holes in each section (12, 14, 16, 18, 20, 22) and end effector (26) are used to provide independent control to each respective section (12, 14, 16, 18, 20, 22) and end effector (26). Each section (12, 14, 16, 18, 20, 22) and end effector (26) is associated with an antagonistic pair of tendons (28). By antagonistic it is meant that tensioning one of the pair of antagonistic tendons will result in movement of a section (12, 14, 16, 18, 20, 22) or end effector (26) in one direction and tensioning of the other of the pair of antagonistic tendons will result in movement of a section (12, 14, 16, 18, 20, 22) or end effector (26) in the other direction.

Each one of a pair of antagonistic tendons (28) is terminated at a section (14, 16, 18, 20, 22) or end effector (26). Termination of tendons (28) is effected by collapsing the tendon holes (12g - for the first section) through the relevant section (14, 16, 18, 20, 22) or end effector (26) to prevent further movement of the tendons (28) relative to that section (14, 16, 18, 20, 22) or end effector (26).

Tendons (28) associated with control of sections (18, 20, 22) located nearer to the end effector (26) pass through the neutral axis of the bending plane of adjacent sections to reduce motion coupling between adjacent sections.

In some embodiments only a selected number of sections are required to be independently controlled. In such embodiments, tendons (28) provide passive control to those sections not associated with a pair of terminated antagonistic tendons (28). Such an embodiment might be used in a surgical instrument used for. cutting tissue where high manual dexterity is not needed. Surgical instruments used for manipulating tissue or using a needle and thread need a greater degree of manual dexterity.

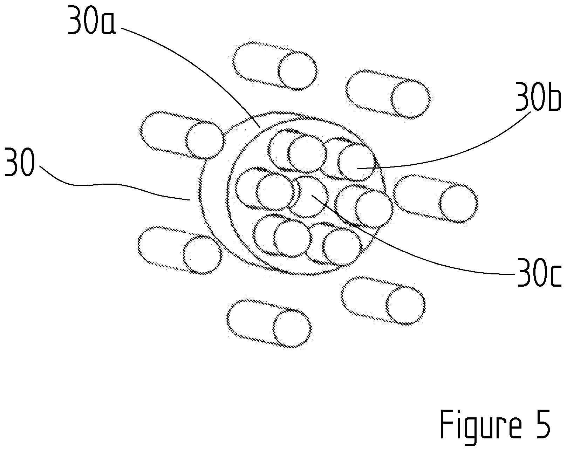

The lumens (12e, 14e, for example) in each section in some embodiments are fitted with a multi-lumen polytetrafluoroethylene (PTFE) catheter (30) as shown in figure 5. The PTFE catheter (30) comprises a generally cylindrical rod (30a) having a plurality of lumens (30b) therethrough surrounding a central lumen (30c). Each of the plurality of lumens (30b) is 40 configured to receive a tendon for independent control of the end effector (26).

The PTFE catheter (30) assists ire keeping the tendons for controlling the end effector passing therethrough as close as possible to the bending axis of the surgical instrument (10) to prevent a joint coupling effect between adjacent hingedly connected components of the
surgical instrument (10). The PTFE catheter (30) additionally assists to reduce friction between adjacent tendons (28) and between tendons (28) and elbow joints.

The tendons for the end effector (26) are shrouded by Bowden cables (28a), i.e. a flexible cable used to transmit mechanical force or energy by the movement of an inner cable relative to a hollow outer cable housing. The PTFE catheter (30) is only needed if the end effector (26) comprises an articulated tool providing a further degree of freedom of positioning such as a grasper or scissors.

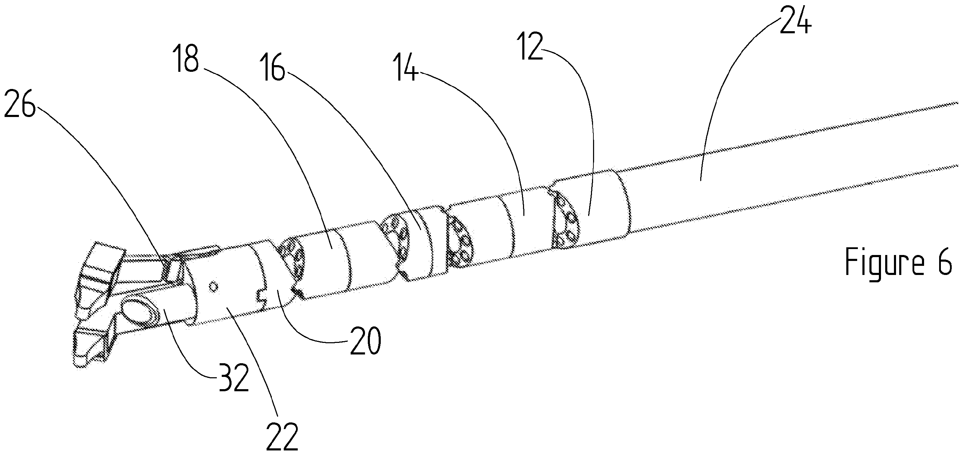

In place of the PTFE catheter (30), the lumen (12e, 14e, for example) through each of the elbow joints can receive a flexible suction and/or irrigation tube (32) as shown in figure 6. The flexible tube (32) is intended for use with either a monopolar knife or bipolar tweezers. Both types of tool require electricity to be supplied to the tip of the end effector (26). In the case of a monopolar tool, electricity is conveyed to the tip of the end effector (26) through the metal structure of the end effector (26). In the case of bipolar tweezers, electricity is conveyed to one side of the tweezers by the metal structure of the end effector (26). An electrical wire conveys electricity from the electrified tweezer side to the other tweezer side which is otherwise electrically isolated from the first side.

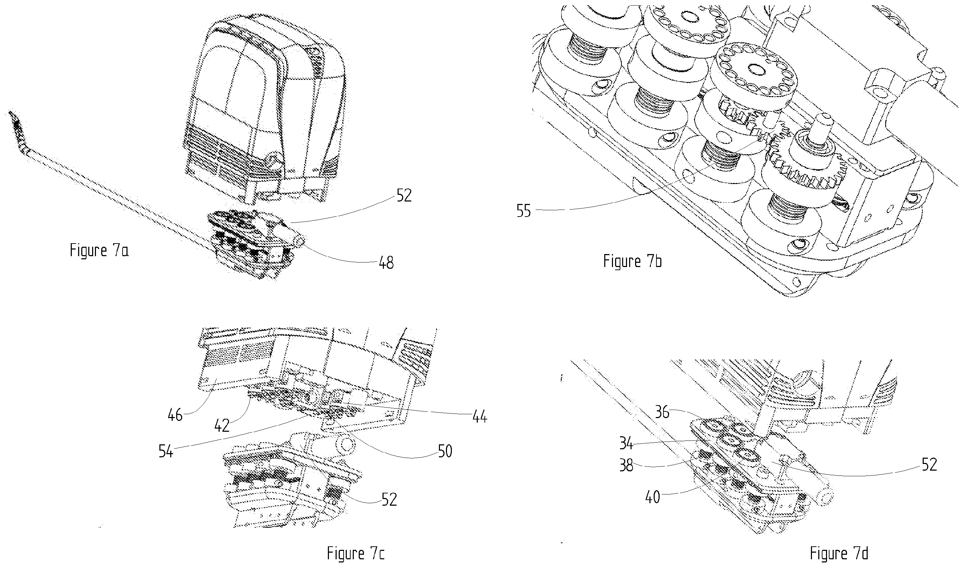

The instrument base (34), as shown in figures 7a to 7d, comprises six motor couplings (36) each associated with respective capstans (38) around which individual tendons (28) are wound. Each motor coupling (36) on the instrument base (34) comprises a plurality of holes (40) for engagement with a plurality of corresponding pins (42) on a corresponding motor coupling (44) on a motor pack (46). Each motor coupling (44) on the motor pack (46) is associated with a respective independently driven motor. Each motor coupling (36) on the instrument base (34) is made from medical grade polyetheretherketon.

Upon attaching the instrument base (34) to the motor pack (46), the motor couplings (36) on the instrument base (34) are each coupled to respective corresponding motor couplings (44) on the motor pack (46) by rotating the motor couplings (36, 44) on either the instrument base (34) or motor pack (44) until the pins (42) on the motor couplings (44) on the motor coupling (46) engage with the holes (40) on the motor couplings (36) on the instrument base (34). Either or both of the motor couplings (36, 44) on the instrument base (34) and/or motor pack (46) are spring loaded to provide a positive engagement between the pins (42) on the motor couplings (44) on the motor pack (46) and the holes (40) on the motor couplings (36) on the instrument base (34). The instrument base (34) is secured to the motor pack (46) by inserting a locking pin (48) through a locking feature (50) on the motor pack (46) and into a corresponding locking feature (52) on the instrument base (34).

Each motor coupling (36) on the instrument base (34) is associated with driving a capstan (38) to wind a tendon (28) for operating a section (12, 14, 16, 18, 20, 22) or end effector (26). An idle gear (55) (as shown in figure 7b) is positioned between two capstans. A gear ratio of 2:1 to between the two capstans reflect the tendon travel difference between the two parallel joints to enable a single motor to drive the two capstans to achieve the desired actuation of the two parallel joints between sections (12, 14, 16). The joints between sections (16, 18, 20) are coupled in the same way by another idle gear on the other side of the instrument base (34).

A translation gear (54) is attached to a motor output shaft directly. The gear (54) drives the instrument and motor pack moving along a rack (not shown) for linear translation.

The end effector (26) can be a grasper, needle driver or scissors, for example and is coupled to the final section (20) of the surgical instrument (10) by way of an end effector (22). The end effector (26) is coupled to the final section (22) of the surgical instrument (10) by way of a hinge arrangement orientated perpendicularly to the hinged coupling between the fourth section (18) and final section (22). The hinged coupling between the final section (22) and end effector (26) is also perpendicular to the hinged coupling between the fifth section (20) and sixth section (22).

Examples of end effector (26) disclosed herein include: i) a wristed grasper - seven degrees of freedom tool with grasper jaws which can be either straight or curved and which is used to manipulate tissue, ii) wristed scissors - seven degrees of freedom wristed tool with scissor blades used to cut tissue with either curved or straight blades, iii) non-wristed scissors - six degrees of freedom tool with scissor blades used to cut tissue with either curved or straight blades, iv) wristed needle driver - seven degrees of freedom tool with straight short jaws having a diamond shaped knurling to grip onto surgical needles, v) non-wristed needle driver - six degrees of freedom tool with straight short jaws having a diamond shaped knurling to grip onto surgical needles, vi) monopolar knife with suction/irrigation - a four degree of freedom multi-functional tool without wrist joint and jaws used for tissue re-section, tissue cauterization, suction of liquid/smoke and irrigation, vii) Bipolar tweezers with suction/irrigation - a five degree of freedom non-wristed multifunctional tool having one moving jaw and used for tissue resection, tissue cauterization, suction of liquid/smoke and irrigation, viii) non-wristed grasper tools.

A monopolar tool, i.e. a knife, can provide electrocautery (tissue cut and cauterization) as well as suction and irrigation. Such a tool is multi-functional and enables a surgeon to excise and cauterise tissue while at the same time removing smoke by way of the suction function. Irrigation is used to wash the wound and suction can again be used to clear the wound from fluids, i.e. blood and saline.

A particular example of end effector (26) is a jawed grasper (400) having a pair of opposed jaws. Each jaw (400a) of the end effector (26) is formed of unitary construction and comprises a gripping surface (400b) defined by the internal surface of an elongate member (400c). The elongate member (400c) further comprises a recess (400d) opposite the gripping surface (400b). The recess (400d) extends longitudinally along the elongate member (400c) and is configured to receive a sensor (402) shaped to correspond with the overall profile of the elongate member (400c). The elongate member (400c) is joined to a mounting boss (400e) defined by two spaced apart plates (400f, 400g) having a gap therebetween. A mounting hole (400h) passes through the mounting boss (400e) for receiving a pivot (not shown).

The sensor (402) has a first insertion portion (402a) and a second insertion portion (402b) which are cooperable with a respective first receiving portion (400i) and second receiving portion (400j) of the elongate member (400c) of the jaw (400a). The sensor (402) can be a force sensor, temperature sensor, tactile sensor, for example.

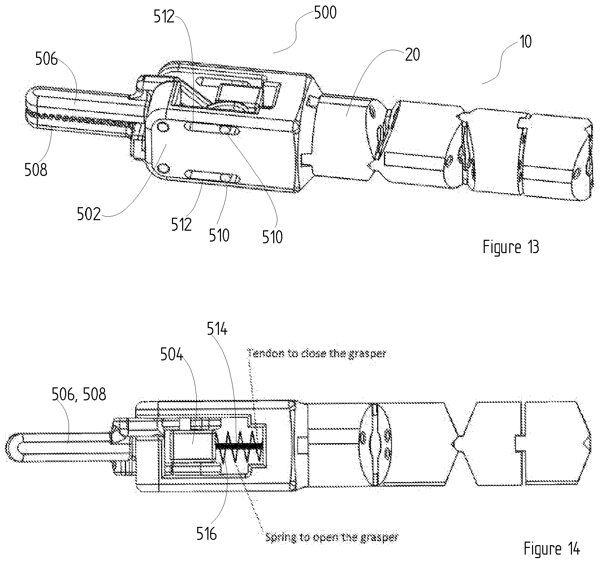

Another example of end effector (26) not according to the invention is a needle driver (500) as illustrated in figures 13 and 14. The needle driver (500) is fixedly coupled to the final section (20) of the surgical instrument (10) by way of a splined connection (20a). The needle driver (500) comprises a body (502) having a mounting arrangement (504) co-operable with each of a pair of opposed grasping jaws (506, 508). The mounting arrangement (504) facilitates pivotal movement of a mounting part of each jaw (506, 508) to permit the jaws (506, 508) to open and close by way of a pin (510) passing through each jaw (506, 508) and the body (502). As shown in figure 13, there are two pins (510), one for each jaw (506, 508), which are spaced apart laterally and positioned adjacent the edge of the body (502) and terminate in a groove (512) on each of opposing sides of the body (502).

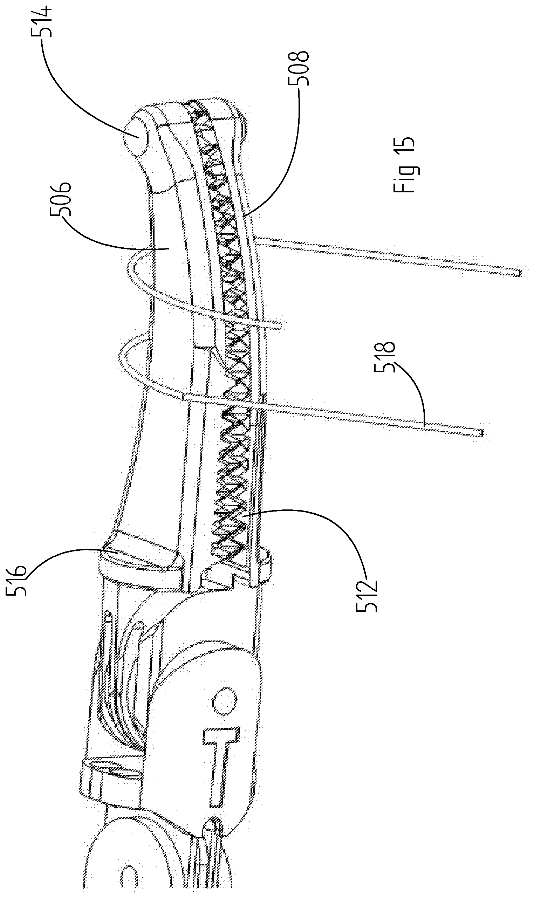

The teeth (512), as better illustrated in figure 15, are triangular shaped and disposed in alternate rows to permit interlocking of the teeth (512) when the jaws (506, 508) are closed. Each tooth (512) has a base that measures 0.25mm, a height of 0.5mm and a width of 0.35mm. The teeth are placed in rows spaced 0.47mm apart. Every row of teeth presents five teeth. Alternating the position of the teeth ensures that the teeth from a first jaw (506) fall between spaced between neighbouring teeth (512) on the second jaw (508). Furthermore, the tip of the needle driver (500) features a nose (514) that is used to retain the thread of a suture (518) during knot tying thus preventing escape of the suture from the jaws (506, 508). The nose (514) comprises a bulbous end at the distal end of each jaw (506, 508). The proximal end of the jaws (506, 508) features a disc (516) having an outer diameter greater than the diameter of the instrument shaft. The disc (516) prevents the suture thread from wrapping around the instrument shaft. The profile of the jaws (506, 508) is rounded in some embodiments.

Movement of the jaws (506, 508) is controlled by a tendon (514) and a spring (516). The jaws (506, 508) are biased in an open position by the spring (516). The spring (514) tension is overcome by tensioning the tendon (514) to close the jaws (506, 508).

The motor pack (46) is selectively mountable to a robotic arm (100) or to a port as described in further detail below.

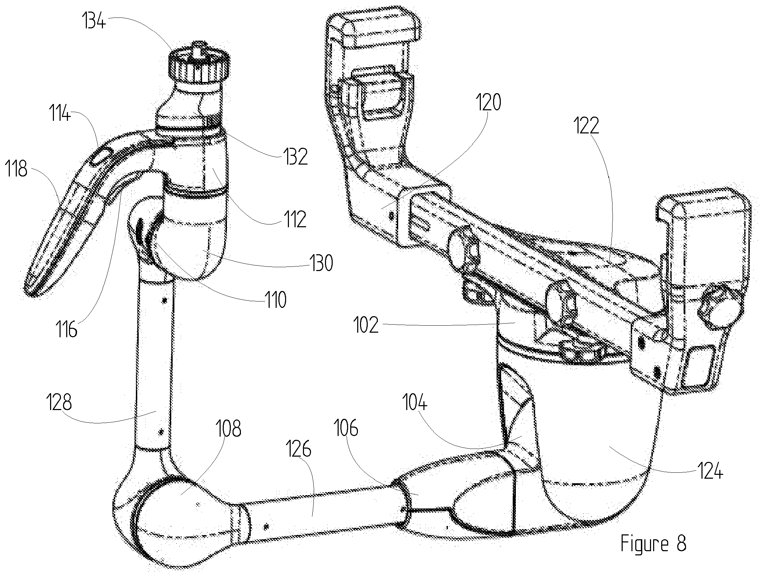

The robotic arm (100), as shown in figure 8, comprises six electromagnetically braked joints (102, 104, 106, 108, 110, 112). Each electromagnetically braked joint (102, 104, 106, 108, 110, 112) comprises an electromagnetic brake and a backlash-free differential drive equipped with an absolute angle joint encoder. The electromagnetic brakes are biased in an on position and releasable by depression of two operation switches (114, 116) located on a handle (118). The robotic arm (100) is mountable to a hospital bed by way of a mounting formation (120) coupled to the robotic arm (100).

The mounting formation (120) is coupled to an anchor (122). The anchor (122) is coupled to a shoulder (124) by way of a first electromagnetically braked joint (102). The anchor (122) provides horizontal rotation relative to the shoulder (124). The shoulder (124) is coupled to a horizontal shaft (126) by way of a second electromagnetically braked joint (104). The shoulder (124) provides pivotal rotation relative to the horizontal shaft (126) in the direction of the longitudinal axis of the horizontal shaft (126). The horizontal shaft (126) extends through a third electromagnetically braked joint (106). The horizontal shaft (126) provides rotational positioning relative to the shoulder (124). The opposite end of the horizontal shaft (126) is coupled to a fourth electromagnetically braked joint (108). The fourth electromagnetically braked joint (108) is coupled to a vertical shaft (128). The vertical shaft (128) provides rotational positioning relative to the horizontal shaft (126). The vertical shaft (128) is coupled at the other end to a fifth electromagnetically braked joint (110). The fifth electromagnetically braked joint (110) is coupled to an elbow (130). The elbow (130) provides rotational positioning around a horizontal axis parallel to the horizontal axis of the horizontal shaft (126). The elbow (130) is coupled to a sixth electromagnetically braked joint (112) at the other end thereof. The sixth electromagnetically braked joint (112) is coupled to the handle (118). The handle is free to rotate around a vertical axis in order to position an adaptor (132) coupled to the handle (118).

The adaptor (132) mounts the motor pack (46) and consequently the surgical instrument (10) to the robotic arm (100).

In use, the robotic arm (100) is mounted to a standard operating table by way of the mounting formation (120) which clamps the robotic arm (100) to the side rails of the standard operating table. The robotic arm (100) and surgical instrument (10) are both electrically powered from a mains supply power outlet through an AC/DC power adaptor. The power supply controls each of the electromagnetically braked joints (102, 104, 106, 108, 110, 112) with electromagnets associated with each being locked in place unless the operation switches (114, 116) on the handle (118) are depressed. Upon depression of both operation switches (114, 116) on the handle (118), all electromagnets are released permitted an operator to manoeuvre the robotic arm (100) through all six electromagnetically braked joints (102, 104, 106, 108, 110, 112). Once the robotic arm (100) is in the desired position the operating switches (114, 116) on the handle (118) are released by the operator and all electromagnets are applied to lock all six electromagnetically braked joints (102, 104, 106, 108, 110, 112). The electromagnets will only be released if both operation switches (114, 116) on the handle (118) are depressed. If only one operation switch (114, 116) is depressed, none of the electromagnets will be released and the operator will not be able to manoeuvre the robotic arm (100) through any of the electromagnetically braked joints (102, 104, 106, 108, 110, 112). This is a safety feature to prevent inadvertent movement of the robotic arm (100).

When the whole arm is locked, the differential driver's output shaft will have a trivial relative rotation to the driver's body if a force is applied to the arm's end-effector. Such rotation can be measured by the joint angle encoder and consequently the torque on the differential drive caused by the force on the end-effector can be calculated considering the stiffness of the differential drive. By taking into account the torque on every joint, the magnitude and direction of the force on the end-effector can be calculated. The combination of an electromagnetic brake with a backlash-free differential drive has the advantage of small footprint and large output torque comparing to the conventional solutions: 1. combination of a motor and a differential drive, in which the motor has much smaller holding torque comparing to the same size brake; 2. only using brake without differential drive, in which the output torque is less and the footprint is larger than our solution.

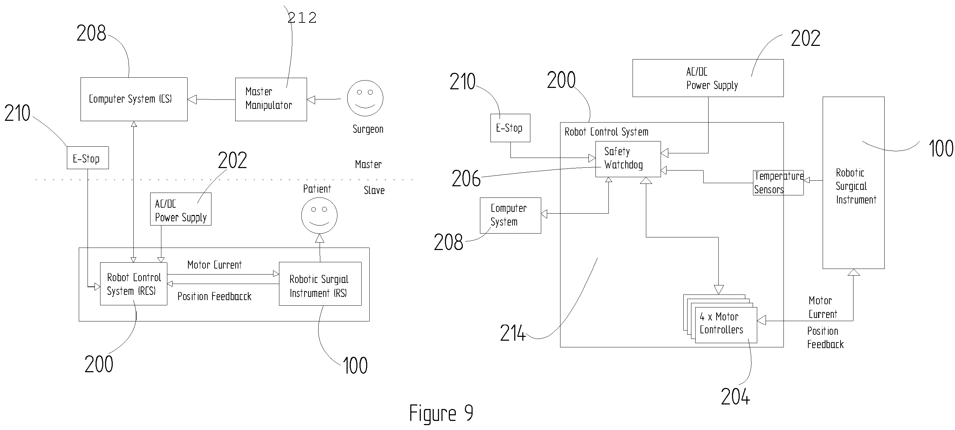

Once a motor pack (46) is mounted to the adaptor (132) and a surgical instrument (10) is coupled to the motor pack (46), power is applied to the motor pack by way of a mains power supply. The motor pack (46) is controlled by a robot control system (200) as illustrated in figure 9.

The robot control system (200) is powered by a separate mains power supply (202) and comprises a plurality of motor controller modules (204), four are shown in figure 9, and a safety watchdog module (206). The safety watchdog (206) is connected between the mains power supply (202) and the plurality of motor controller modules (204). The robot control system (200) is connected between the robotic surgical instrument (100) and a computer system (208). The robot control system (200) is further provided with an emergency stop button (210) for cutting all power to the robot control system (200) and thus the surgical instrument (10). A master manipulator (212) is connected to the computer system (208). The computer system (208) interprets movement of the master manipulator (212) to determine the desired action of the surgical instrument (10) and sends appropriate instructions to the robot control system (200) via a RS-485 bus to drive the plurality of motor controllers (204).

The safety watchdog module (206) monitors a number of parameters of the robot control system (200) and/or surgical instrument (10) such as temperature and motor current for example. If the safety watchdog module (206) detects that a parameter has deviated from a pre-determined range or exceeded a pre-determined threshold, the safety watchdog module (206) will cut all power to the motor controller modules (204) to prevent erroneous operation and/or damage/injury to a patient. The safety watchdog module (206) also listens to communication between the computer system (208) and robot control system (200) and between the robot control system (200) and the surgical instrument (10). If instructions are detected that fall outside of accepted operating parameters the safety watchdog module (206) will cut all power to the robot control system (200) to prevent erroneous operation and/or damage/injury to a patient.

The safety watchdog module (206) is a modular component that plugs into a motherboard (214). Each motor controller module (204) is also a modular component that plugs into the motherboard (214). Each motor controller module (204) can control up to two motors and the motherboard (214) can accommodate up to four motor controller modules (204) allowing connection of up to eight motors for driving the robotic surgical instrument (100). This disclosure is not intended to be limiting; other embodiments may be capable of accommodating further motor control modules and each motor control module may be capable of controlling one, two or more motors.

The adaptor (132) includes an electrical connector (134) which can supply power and control signals via the internal wiring of the robotic arm (100). The motor pack (46) can be powered and controlled via either the electrical connection (134) or independent cables.

To ensure that the surgical instrument (10) is only movable within a pre-defined boundary, a three dimensional boundary space or spatial threshold is defined prior to commencing surgery. The three dimensional boundary space is defined by moving the robotic arm (100) through a series of spatial points and recording each spatial point as a boundary point. The robotic arm during surgery is only permitted to move within the three dimensional boundary and is automatically locked should it hit, or in some instances approach, the three dimensional boundary.

Once movement of the robotic arm (100) is locked, there are a number of ways that it can be unlocked to resume surgery. Two examples will now be described.

In a first example, the robotic arm (100) comprises a rotary encoder that monitors every movement of each of the electromagnetically braked joints (102, 104, 106, 108, 112) and the surgical instrument end effector (22). Each movement is recorded as a data point relative to a respective origin point. The rotary encoder permits each electromagnetically braked joint (102, 104, 106, 108, 110, 112) and thus the surgical instrument end effector (22) to move through each data point in reverse. Once each data point is determined as being equal to a respective origin point, each of the electromagnetically braked joints (102, 104, 106, 108, 110, 112) is fully released.

In a second example, force detection means are associated with each of the electromagnetically braked joints (102, 104, 106, 108, 110, 112). A processor equates a force applied by a surgeon to a master manipulator (212) to direction and unlocks the electromagnetically braked joints (102, 104, 106, 108, 110, 112) if it is determined that all of the electro magnetically braked joints (102, 104, 106, 108, 110, 112) and the surgical instrument end effector (22) would be moved away from the three-dimensional boundary. If it is determined that one or more of the electromagnetically braked joints (102, 104, 106, 108, 110, 112) and/or the end effector (22) would be moved towards or cross the three-dimensional boundary, each of the electromagnetically braked joints (102, 104, 106, 108, 110, 112) would remain locked and movement would be resisted.

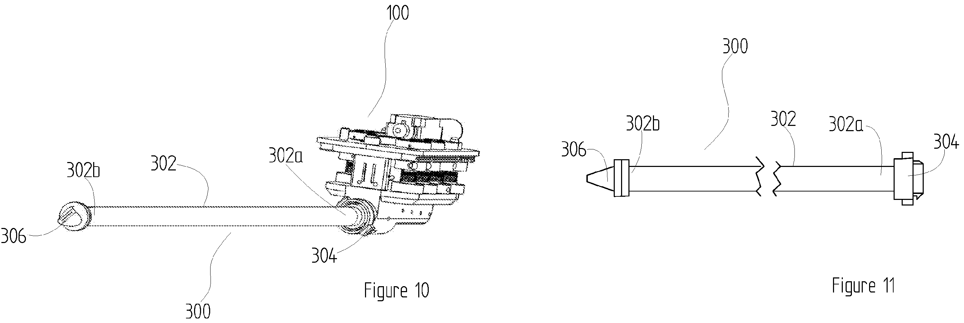

Referring to figures 10 and 11, a protective sleeve (300) for use with surgical instruments (10) of embodiments of the invention is shown. The protective sleeve (300) comprises an elongate sheath (302) that has a first end (302a) and a second end (302b). The elongate sheath is formed from a thin plastic material and is flexible and compressible. The first end (302a) of the elongate sheath (302) is attachable to a surgical instrument by way of an attachment interface (304). The attachment interface may comprise a locking means such as a twist locking mechanism or snap fit interface or may be magnetic. The second end (302b) of the elongate sheath (302) defines an interface for attachment of an end closure (306) such as a duckbill valve or other type of suitable valve. The end closure (306) may be attached to the second end (302b) of the elongate sheath (302) by way of a locking means or magnetic attachment, for example.

In use, the end effector end of a surgical instrument (10) is inserted into the protective sleeve (300) after sterilisation. The protective sleeve (300) is attached to the surgical instrument (10) by way of the attachment interface (304). The surgical instrument (10) is inserted into a lumen of a port immediately prior to start of surgery. In embodiments utilising a magnet to attach the closure means (306) to the second end (302b) of the protective sleeve (300), the magnet is used to align the surgical instrument (10) with the lumen of the port. The closure means (306) is sized appropriately to enable it to extend through the lumen of the port. As the surgical instrument (10) is advanced, the surgical instrument (10) penetrates through the valve (306) and the protective sleeve (300) is compressed within the port to expose the surgical instrument (10).

Upon conclusion of surgery, the surgical instrument (100) is withdrawn from the patient and through the port into the protective sleeve (300). The surgical instrument passes back through the valve which closes once the surgical instrument is again fully enclosed by the protective sleeve (300). Prior to re-use, the surgical instrument is sterilised through autoclave, gas or radiation treatment and a new protective sleeve (300) is fitted to the surgical instrument (100). The used protective sleeve (300) is discarded as hazardous waste after surgery.

## Claims

1. A surgical instrument (10) comprising: a rigid shaft (24), at least one elbow joint, itself comprising at least three elbow joints, hingedly coupled to the rigid shaft (24) and a wrist joint coupled to the at least one elbow joint, wherein the wrist joint is configured to provide a first degree of freedom of movement and a second degree of freedom of movement, wherein the second degree of freedom of movement is substantially perpendicular to the first degree of freedom of movement;
wherein two of the at least three elbow joints are arranged to provide a hinged motion in the same direction and a third elbow joint is arranged to provide a hinged motion in a different direction to the other elbow joints and wherein at least two adjacent elbow joints are coupled together such that the at least two adjacent elbow joints move in unison; and
wherein the wrist joint comprises first and second sections (20, 22) and an end effector (26) having first and second jaws, the first section (20) defining a first hinged joint (21) with the second section (22) so that the second section (22) is hingedly moveable relative to the first section (20) and the at least one elbow joint to provide the first degree of freedom of movement of the wrist joint, the second section (22) defining a second hinged joint (27) with each of the first and second jaws of the end effector (26) so that each jaw is hingedly moveable relative to the first and second sections (20, 22) and the at least one elbow joint, the second hinged joint (27) being arranged perpendicular to the first hinged joint (21) so as to provide the second degree of freedom of movement of the wrist joint.

2. A surgical instrument (10) according to claim 1, wherein the at least one elbow joint comprises a fourth elbow joint.

3. A surgical instrument (10) according claim 2, wherein the fourth elbow joint is arranged to provide a hinged motion in substantially the same direction as the third elbow joint.

4. A surgical instrument (10) according to any preceding claim, further comprising at least one further elbow joint movable independently of any other elbow joint.

5. A surgical instrument (10) according to claim 1, wherein the at least one elbow joint and the wrist joint are independently movable by way of tendon drive means.

6. A surgical instrument (10) according to any preceding claim, wherein the at least one elbow joint is movable through a range of motion between zero and sixty degrees.

7. A surgical instrument (10) according to any preceding claim, wherein the first degree of freedom of movement provides a first hinged joint (21) configured to permit pivoting of the wrist joint through an angular range of one hundred and eighty degrees relative to the at least one elbow joint.

8. A surgical instrument (10) according to any preceding claim, wherein the second degree of freedom of movement provides a second hinged joint (27) configured to permit pivoting of the wrist joint perpendicular to the first hinged joint through an angular range of between zero and sixty degrees relative to the at least one elbow joint.

9. A surgical instrument (10) according to any preceding claim further comprising an end effector (26).

10. A surgical instrument (10) according to claim 9, wherein the end effector (26) is a monopolar end effector or a bipolar end effector.

11. A surgical instrument (10) according to claim 9 or claim 10, wherein the end effector (26) is movable relative to the wrist joint by tendon drive means.

12. A surgical instrument (10) according to claim 11, wherein the tendon drive means are shrouded by Bowden cables.

13. A surgical instrument (10) according to any of claims 9 to 12, wherein the end effector (26) is coupled to the at least one elbow joint, wherein the rigid shaft (24) and the at least one elbow joint define a continuous lumen therethrough, the continuous lumen receiving an auxiliary end effector (32) or providing irrigation or suction functionality.

14. A surgical instrument (10) according to claim 13 further comprising a multi lumen insert positioned within the continuous lumen, the multi lumen insert (30) comprising a plurality of lumens (30b), wherein one or more of the plurality of lumens is configured to receive a respective tendon (28).

15. A surgical instrument (10) according to any preceding claim further comprising a protective sleeve (300), the protective sleeve comprising: an elongate flexible sheath (302) having a first end (302a) and a second end (302b), wherein the first end (302a) comprises an attachment means (304) for attachment of the protective sleeve to the surgical instrument and wherein the second end comprises a closure means (306).

16. A surgical instrument (10) according to claim 15, wherein the attachment means (304) comprises a locking means having a first part located at the first end of the protective sleeve and a second part forming part of a robotic surgical system.

17. A surgical instrument (10) according to claim 16, wherein the attachment means (304) comprises magnetic means having a first part located at the first end (302a) of the protective sleeve (300) and a second part forming part of a robotic surgical system.

18. A surgical instrument (10) according to any of claims 15 to 17, wherein the flexible sheath (302) is compressible.

19. A surgical instrument (10) according to any of claims 15 to 18, wherein the closure means (306) is a valve.

20. A surgical instrument (10) according to claim 19, wherein the valve is attached to the second end of the elongate sheath by way of a magnetic attachment means.

## Patentansprüche

1. Chirurgisches Instrument (10), umfassend: einen starren Schaft (24), mindestens ein Ellenbogengelenk, das selbst mindestens drei Ellenbogengelenke umfasst, die gelenkig mit dem starren Schaft (24) verbunden sind, und ein Handgelenk, das mit dem mindestens einen Ellenbogengelenk verbunden ist, wobei das Handgelenk so konfiguriert ist, dass es einen ersten Bewegungsfreiheitsgrad und einen zweiten Bewegungsfreiheitsgrad bietet, wobei der zweite Bewegungsfreiheitsgrad im Wesentlichen senkrecht zum ersten Bewegungsfreiheitsgrad verläuft;
wobei zwei der mindestens drei Ellenbogengelenke so angeordnet sind, dass sie eine Scharnierbewegung in die gleiche Richtung ermöglichen, und ein drittes Ellenbogengelenk so angeordnet ist, dass es eine Scharnierbewegung in eine andere Richtung als die anderen Ellenbogengelenke ermöglicht, und wobei mindestens zwei benachbarte Ellenbogengelenke so miteinander gekoppelt sind, dass sich die mindestens zwei benachbarten Ellenbogengelenke im Gleichklang bewegen; und
wobei das Handgelenk erste und zweite Abschnitte (20, 22) und einen Endeffektor (26) mit ersten und zweiten Backen umfasst, wobei der erste Abschnitt (20) mit dem zweiten Abschnitt (22) eine erste Scharnierverbindung (21) bildet, so dass der zweite Abschnitt (22) relativ zum ersten Abschnitt (20) und dem mindestens einen Ellenbogengelenk gelenkig beweglich ist, um den ersten Bewegungsfreiheitsgrad des Handgelenks bereitzustellen, wobei der zweite Abschnitt (22) eine zweite Scharnierverbindung (27) mit jeder der ersten und zweiten Backen des Endeffektors (26) bildet, so dass jede Backe relativ zum ersten und zweiten Abschnitt (20, 22) und dem mindestens einen Ellenbogengelenk gelenkig beweglich ist, wobei das zweite Scharniergelenk (27) senkrecht zum ersten Scharniergelenk (21) angeordnet ist, um den zweiten Bewegungsfreiheitsgrad des Handgelenks bereitzustellen.

2. Chirurgisches Instrument (10) nach Anspruch 1, wobei das mindestens eine Ellenbogengelenk ein viertes Ellenbogengelenk umfasst.

3. Chirurgisches Instrument (10) nach Anspruch 2, wobei das vierte Ellenbogengelenk angeordnet ist, um eine Scharnierbewegung im Wesentlichen in die gleiche Richtung wie das dritte Ellenbogengelenk bereitzustellen.

4. Chirurgisches Instrument (10) nach einem der vorhergehenden Ansprüche, ferner umfassend mindestens ein weiteres Ellenbogengelenk, das unabhängig von jedem anderen Ellenbogengelenk beweglich ist.

5. Chirurgisches Instrument (10) nach Anspruch 1, wobei das mindestens eine Ellenbogengelenk und das Handgelenk über Sehnenantriebsmittel unabhängig voneinander bewegbar sind.

6. Chirurgisches Instrument (10) nach einem der vorhergehenden Ansprüche, wobei das mindestens eine Ellenbogengelenk über einen Bewegungsbereich zwischen null und sechzig Grad beweglich ist.

7. Chirurgisches Instrument (10) nach einem der vorhergehenden Ansprüche, wobei der erste Bewegungsfreiheitsgrad ein erstes Scharniergelenk (21) bereitstellt, das konfiguriert ist, um eine Drehung des Handgelenks um einen Winkelbereich von einhundertachtzig Grad relativ zu dem mindestens einen Ellenbogengelenk zu ermöglichen.

8. Chirurgisches Instrument (10) nach einem der vorhergehenden Ansprüche, wobei der zweite Bewegungsfreiheitsgrad ein zweites Scharniergelenk (27) bereitstellt, das konfiguriert ist, um eine Drehung des Handgelenks senkrecht zum ersten Scharniergelenk über einen Winkelbereich zwischen null und sechzig Grad relativ zu dem mindestens einen Ellenbogengelenk zu ermöglichen.

9. Chirurgisches Instrument (10) nach einem der vorhergehenden Ansprüche, das ferner einen Endeffektor (26) umfasst.

10. Chirurgisches Instrument (10) nach Anspruch 9, wobei der Endeffektor (26) ein monopolarer Endeffektor oder ein bipolarer Endeffektor ist.

11. Chirurgisches Instrument (10) nach Anspruch 9 oder Anspruch 10, wobei der Endeffektor (26) durch Sehnenantriebsmittel relativ zum Handgelenk beweglich ist.

12. Chirurgisches Instrument (10) nach Anspruch 11, wobei die Sehnenantriebsmittel durch Bowdenzüge ummantelt sind.

13. Chirurgisches Instrument (10) nach einem der Ansprüche 9 bis 12, wobei der Endeffektor (26) mit dem mindestens einen Ellenbogengelenk gekoppelt ist, wobei der starre Schaft (24) und das mindestens eine Ellenbogengelenk einen durchgehenden Hohlraum definieren, wobei der durchgehende Hohlraum einen Hilfsendeffektor (32) aufnimmt oder eine Spül- oder Saugfunktion bereitstellt.

14. Chirurgisches Instrument (10) nach Anspruch 13, ferner umfassend einen Multilumen-Einsatz, der innerhalb des kontinuierlichen Lumens positioniert ist, wobei der Multilumen-Einsatz (30) eine Vielzahl von Lumen (30b) umfasst, wobei eines oder mehrere der Vielzahl von Lumen so konfiguriert sind, dass sie eine entsprechende Sehne (28) aufnehmen.

15. Chirurgisches Instrument (10) nach einem der vorhergehenden Ansprüche, ferner umfassend eine Schutzhülle (300), wobei die Schutzhülle Folgendes umfasst: eine längliche flexible Hülle (302) mit einem ersten Ende (302a) und einem zweiten Ende (302b), wobei das erste Ende (302a) ein Befestigungsmittel (304) zur Befestigung der Schutzhülle am chirurgischen Instrument umfasst und wobei das zweite Ende ein Verschlussmittel (306) umfasst.

16. Chirurgisches Instrument (10) nach Anspruch 15, wobei das Befestigungsmittel (304) ein Verriegelungsmittel umfasst, das einen ersten Teil, der sich am ersten Ende der Schutzhülle befindet, und einen zweiten Teil aufweist, der Teil eines chirurgischen Robotersystems ist.

17. Chirurgisches Instrument (10) nach Anspruch 16, wobei das Befestigungsmittel (304) magnetische Mittel umfasst, die einen ersten Teil, der sich am ersten Ende (302a) der Schutzhülle (300) befindet, und einen zweiten Teil aufweist, der Teil eines chirurgischen Robotersystems ist.

18. Chirurgisches Instrument (10) nach einem der Ansprüche 15 bis 17, wobei die flexible Hülle (302) komprimierbar ist.

19. Chirurgisches Instrument (10) nach einem der Ansprüche 15 bis 18, wobei das Verschlussmittel (306) ein Ventil ist.

20. Chirurgisches Instrument (10) nach Anspruch 19, wobei das Ventil über magnetische Befestigungsmittel am zweiten Ende der länglichen Hülle befestigt ist.

## Revendications

1. Instrument chirurgical (10) comprenant : une tige rigide (24), au moins une articulation de coude, comprenant elle-même au moins trois articulations de coude, couplée de manière articulée à la tige rigide (24) et une articulation de poignet couplée à l'au moins une articulation de coude, dans lequel l'articulation de poignet est conçue pour fournir un premier degré de liberté de mouvement et un second degré de liberté de mouvement, dans lequel le second degré de liberté de mouvement est sensiblement perpendiculaire au premier degré de liberté de mouvement ;
dans lequel deux des au moins trois articulations de coude sont agencées pour fournir un mouvement articulé dans la même direction et une troisième articulation de coude est agencée pour fournir un mouvement articulé dans une direction différente par rapport aux autres articulations de coude et dans lequel au moins deux articulations de coude adjacentes sont couplées ensemble de sorte que lesdites au moins deux articulations de coude adjacentes se déplacent à l'unisson ; et
dans lequel l'articulation de poignet comprend des première et seconde sections (20, 22) et un effecteur terminal (26) présentant des première et seconde mâchoires, la première section (20) définissant une première articulation articulée (21) avec la seconde section (22) de sorte que la seconde section (22) est mobile de manière articulée par rapport à la première section (20) et à l'au moins une articulation de coude pour fournir le premier degré de liberté de mouvement de l'articulation de poignet, la seconde section (22) définissant une deuxième articulation articulée (27) avec chacune des première et seconde mâchoires de l'effecteur terminal (26) de sorte que chaque mâchoire est mobile de manière articulée par rapport aux première et seconde sections (20, 22) et l'au moins une articulation de coude, la deuxième articulation articulée (27) étant disposée perpendiculairement à la première articulation articulée (21) de manière à fournir le second degré de liberté de mouvement de l'articulation de poignet.

2. Instrument chirurgical (10) selon la revendication 1, dans lequel l'au moins une articulation de coude comprend une quatrième articulation de coude.

3. Instrument chirurgical (10) selon la revendication 2, dans lequel la quatrième articulation de coude est agencée pour fournir un mouvement articulé sensiblement dans la même direction que la troisième articulation de coude.

4. Instrument chirurgical (10) selon une quelconque revendication précédente, comprenant en outre au moins une autre articulation de coude mobile indépendamment de tout autre articulation de coude.

5. Instrument chirurgical (10) selon la revendication 1, dans lequel l'au moins une articulation de coude et l'articulation de poignet sont mobiles indépendamment grâce à un moyen d'entraînement de tendon.

6. Instrument chirurgical (10) selon une quelconque revendication précédente, dans lequel l'au moins une articulation de coude est mobile sur une plage de mouvement comprise entre zéro et soixante degrés.

7. Instrument chirurgical (10) selon une quelconque revendication précédente, dans lequel le premier degré de liberté de mouvement fournit une première articulation articulée (21) conçue pour permettre le pivotement de l'articulation de poignet sur une plage angulaire de cent quatre-vingts degrés par rapport à l'au moins une articulation de coude.

8. Instrument chirurgical (10) selon une quelconque revendication précédente, dans lequel le second degré de liberté de mouvement fournit une deuxième articulation articulée (27) conçue pour permettre le pivotement de l'articulation de poignet perpendiculairement à la première articulation articulée sur une plage angulaire comprise entre zéro et soixante degrés par rapport à l'au moins une articulation de coude.

9. Instrument chirurgical (10) selon une quelconque revendication précédente, comprenant en outre un effecteur terminal (26).

10. Instrument chirurgical (10) selon la revendication 9, dans lequel l'effecteur terminal (26) est un effecteur terminal monopolaire ou un effecteur terminal bipolaire.

11. Instrument chirurgical (10) selon la revendication 9 ou la revendication 10, dans lequel l'effecteur terminal (26) est mobile par rapport à l'articulation de poignet grâce à un moyen d'entraînement de tendon.

12. Instrument chirurgical (10) selon la revendication 11, dans lequel le moyen d'entraînement de tendon est enveloppé par des câbles Bowden.

13. Instrument chirurgical (10) selon l'une quelconque des revendications 9 à 12, dans lequel l'effecteur terminal (26) est couplé à l'au moins une articulation de coude, dans lequel la tige rigide (24) et l'au moins une articulation de coude définissent une lumière continue à travers celui-ci, la lumière continue recevant un effecteur terminal auxiliaire (32) ou assurant une fonctionnalité d'irrigation ou d'aspiration.

14. Instrument chirurgical (10) selon la revendication 13, comprenant en outre un insert à lumières multiples positionné à l'intérieur de la lumière continue, l'insert à lumières multiples (30) comprenant une pluralité de lumières (30b), dans lequel une ou plusieurs lumières de la pluralité de lumières sont conçues pour recevoir un tendon respectif (28).

15. Instrument chirurgical (10) selon une quelconque revendication précédente, comprenant en outre un manchon protecteur (300), le manchon protecteur comprenant : une gaine flexible allongée (302) présentant une première extrémité (302a) et une seconde extrémité (302b), dans lequel la première extrémité (302a) comprend un moyen de fixation (304) pour fixer le manchon de protection à l'instrument chirurgical et dans lequel la seconde extrémité comprend un moyen de fermeture (306).

16. Instrument chirurgical (10) selon la revendication 15, dans lequel le moyen de fixation (304) comprend un moyen de verrouillage présentant une première partie située au niveau de la première extrémité du manchon de protection et une seconde partie faisant partie d'un système chirurgical robotisé.

17. Instrument chirurgical (10) selon la revendication 16, dans lequel le moyen de fixation (304) comprend des moyens magnétiques présentant une première partie située au niveau de la première extrémité (302a) du manchon de protection (300) et une seconde partie faisant partie d'un système chirurgical robotisé.

18. Instrument chirurgical (10) selon l'une quelconque des revendications 15 à 17, dans lequel la gaine flexible (302) est compressible.

19. Instrument chirurgical (10) selon l'une quelconque des revendications 15 à 18, dans lequel le moyen de fermeture (306) est une valve.

20. Instrument chirurgical (10) selon la revendication 19, dans lequel la valve est fixée à la seconde extrémité de la gaine allongée grâce à un moyen de fixation magnétique.
